# EUROPÄISCHE PATENTSCHRIFT

(11) **EP 0 934 082 B1**
(45) Veröffentlichungstag und Bekanntmachung des Hinweises auf die Patenterteilung: **17.07.2002**
(21) Anmeldenummer: 97948768.3
(22) Anmeldetag: 21.10.1997
(51) Int. Cl.: A61K 47/48, A61K 9/00

(54) **VERFAHREN ZUR HERSTELLUNG VON BIOLOGISCH AKTIVEN POLYMERNANOPARTIKEL-NUCLEINSÄURE-KONJUGATEN**
PROCESS FOR PRODUCING BIOLOGICALLY ACTIVE POLYMER NANOPARTICLE-NUCLEIC ACID CONJUGATES
PROCEDE DE PRODUCTION DE CONJUGUES BIOLOGIQUEMENT ACTIFS D'ACIDES NUCLEIQUES ET DE NANOPARTICULES POLYMERES

(30) Priorität: 23.10.1996 DE 19643738
(43) Veröffentlichungstag der Anmeldung: 11.08.1999
(73) Patentinhaber: SKW Trostberg Aktiengesellschaft, 83308 Trostberg (DE)
(72) Erfinder: BAYER, Ernst, D-72076 Tübingen (DE); FRITZ, Hans, D-72072 Tübingen (DE); MAIER, Martin, D-72074 Tübingen (DE)
(74) Vertreter: HOFFMANN - EITLE
(86) Internationale Anmeldenummer: EP9705790
(87) Internationale Veröffentlichungsnummer: WO9817317

(56) Entgegenhaltungen:
- SUGIYAMA, KAZUO ET AL: "Adsorption of protein on the surface of thermosensitive poly(methyl methacrylate) microspheres modified with the N-(2- hydroxypropyl)methacrylamide and 2-(methacryloyloxy)ethyl phosphorylcholine moieties" J. POLYM. SCI., PART A: POLYM. CHEM. (1997), 35(16), 3349-3357 CODEN: JPACEC;ISSN: 0887-624X, 1997, XP002066323

## Beschreibung

Die vorliegende Erfindung betrifft ein Verfahren zur Herstellung von biologisch aktiven Polymernanopartikel-Nucleinsäure-Konjugaten und deren Verwendung zum Gentransfer bzw. zur Kontrolle der Genexpression.

Die Verwendung von Nucleinsäuren und Nucleinsäure-Derivaten, wie z.B. von synthetisch hergestellten Oligodesoxynucleotiden und deren Derivaten zur Steuerung der Genexpression (Antisense-Strategie) oder von DNA-Fragmenten und Plasmiden zum Gentransfer, machen einen wirksamen Transport durch zelluläre Membranen erforderlich. Des weiteren sollten die Nucleinsäuren gegen enzymatischen Abbau geschützt sein und in ausreichender Konzentration ihren Zielort in der Zelle (Zellkern, mRNA im Zytoplasma) erreichen, um die gewünschte Wirkung zu gewährleisten.

Kolloidale Trägersysteme werden seit langem für den Transport von biologisch bzw. therapeutisch wirksamen Substanzen verwendet, wobei an das Trägersystem folgende Anforderungen gestellt werden:
- kleine Partikelgröße, die deutlich unterhalb der Größe von Zellstrukturen (Durchmesser kleiner als 1 µm) liegt
- hohe Beladungskapazität
- niedrige Toxizität
- Möglichkeit zur Oberflächenmodifizierung
- Steuerung des Zielortes durch Variation von Größe bzw. Oberflächeneigenschaften
- kontrollierte Freisetzung der adsorptiv gebundenen Substanzen
- keine Nebenwirkungen durch Trägermaterial, Abbauprodukte oder Hilfsstoffe

Der Einsatz von synthetisch hergestellten Polymerpartikeln im nm-Bereich zur adsorptiven Anbindung von Nucleinsäuren ist bspw. in der FR-A 2,649,321 beschrieben. Hierbei werden Poly(alkylcyanoacrylate) durch die adsorptive Anbindung von niedermolekularen, hydrophoben Kationen mit einer positiven Oberflächenladung versehen, um eine Anlagerung von negativ geladenen Oligodesoxyribonucleotiden über ionische Wechselwirkungen zu ermöglichen. Nachteilig bei diesem System ist die Tatsache, daß die rein adsorptive Bindung niedermolekularer Kationen wenig stabil ist und sowohl die verwendeten hydrophoben Kationen, als auch die Abbauprodukte des Polymergrundkörpers toxische Eigenschaften aufweisen können.

Gemäß der EP-A 430 517 wird die Herstellung von Biomosaikpolymeren in Form von Membranen, Filmen oder Partikeln mittels Emulsionspolymerisation beschrieben, wobei die Polymerisation im wesentlichen in Gegenwart einer oberflächenaktiven Substanz (ionisches oder nichtionisches Tensid) sowie einer biologisch aktiven Substanz (wie z.B. Nucleinsäuren) erfolgt und das biologisch aktive Material irreversibel in den Polymergrundkörper einpolymerisiert bzw. an dessen Oberfläche gebunden wird.

Schließlich ist aus der WO 96/24 377 eine partikuläre Arzneiform bekannt, die aus kationischen Polymernanopartikeln und Peptiden bzw. modifizierten oder nichtmodifizierten Nucleinsäuren bestehen. Als Polymergrundkörper fungieren hierbei Partikelmischpolymere auf Basis von Acrylsäuren, Acrylsäureestern sowie Methacrylsäureestern. Die Einführung der zur Anbindung der Wirkstoffe erforderlichen funktionellen kationischen Gruppen erfolgt durch den Einsatz von entsprechend modifizierten Comonomeren, die protonierbare bzw. alkylierbare Aminfunktionen besitzen. Diese ladungstragenden Monomerbausteine werden in beliebigen Mischungsverhältnissen mit dem entsprechend unmodifizierten Monomer in einer radikalischen oder ionischen Polymerisationsreaktion umgesetzt. Ganz abgesehen davon, daß diese Monomerbausteine aufwendig herzustellen sind, treten aufgrund der vorhandenen Unterschiede in der Löslichkeit und Reaktivität dieser Monomere bei der Herstellung dieser Mischpolymere häufig inhomogene Produkte auf.

Der vorliegenden Erfindung lag daher die Aufgabe zugrunde, ein Verfahren zur Herstellung von Polymernanopartikel-Nucleinsäure-Konjugaten zu entwickeln, welches die genannten Nachteile entsprechend dem Stand der Technik nicht aufweist, sondern in einfacher, kostengünstiger und gut reproduzierbarer Weise die Herstellung homogener Polymernanopartikel-Nucleinsäure-Konjugate ermöglicht, die eine ausreichende Stabilität in biologischen Medien aufweisen und die sich durch eine hohe Effizienz beim Transport durch zelluläre Membranen auszeichnen.

Diese Aufgabe wurde erfindungsgemäß dadurch gelöst, daß man eine Polymerisation von vinylischen Monomeren mit einer geringen Wasserlöslichkeit in Gegenwart von kationischen Radikalstartern in Form einer emulgatorfreien Emulsionspolymerisation durchführt und anschließend die entstehenden Polymersuspensionen mit den Nucleinsäuren umsetzt.

Es hat sich nämlich überraschenderweise gezeigt, daß die so erzeugten Materialien sich durch eine hohe Nucleinsäure-Beladung sowie durch eine ausreichende Stabilität gegen enzymatischen Abbau auszeichnen. Außerdem können diese Konjugate durch viele Parameter in ihren Eigenschaften variiert werden, wie z. B. Polymergrundkörper, Partikelgröße, Oberflächenmodifizierung, Nucleinsäuremodifizierung etc., was ebenfalls nicht vorhersehbar war. Von den Konjugaten gemäß der WO 96/24 377 sind die erfindungsgemäßen Konjugate dadurch verschieden, daß sie kationische Gruppen praktisch ausschließlich an den Kettenenden der jeweiligen Polymerketten, aus denen das Polymer-Nanopartikel besteht, enthalten, nicht aber im Inneren der Polymerkette.

Das Verfahren entsprechend der vorliegenden Erfindung umfaßt also in der Regel zwei Stufen. In der ersten Stufe erfolgt die Herstellung des Polymergrundkörpers in an sich bekannter Weise, indem man vinylische Monomere in wäßrigem Dispersionsmedium einer emulgatorfreien Emulsionspolymerisation unterwirft. Die vinylischen Monomere sollten hierbei eine geringe Wasserlöslichkeit aufweisen, die vorzugweise < 20 g/l betragen sollte. Beispiele für solche vinylische Monomere sind Styrol, Acrylsäure- oder Methacrylsäure- Derivate. Bevorzugte (Meth)acrylsäure-Derivate sind Alkyl(meth)acrylate (mit einem Alkylrest von 1 bis 8 C-Atomen) sowie N-Alkyl- oder Dialkylacrylamide, wie z. B. N-Butylmethylacrylamid, N-Isobutylmethylacrylamid oder N-Octylmethylacrylamid.

Es ist als erfindungswesentlich anzusehen, daß die Emulsionspolymerisation, die vorzugsweise bei Temperaturen von 20 bis 100 °C durchgeführt wird, ohne den Zusatz von Emulgatoren erfolgt und daß die Oberflächenladung des Polymergrundkörpers allein durch die Verwendung ionischer Radikalstarter induziert wird. Hierbei haben sich vor allem kationische Initiatoren mit basischen Endgruppen, wie z. B. 2,2'-Azobis(2-amidinopropan)dihydrochlorid (AIBA) oder 2,2'-Azobis(2-(2-imidazolin-2-yl)propan)dihydrochlorid (AIBI) bewährt.

Aufgrund der Tatsache, daß keine zusätzlichen Fremdstoffe wie Emulgatoren oder Stabilisatoren dem Ansatz hinzugefügt werden, ist eine aufwendige Abtrennung dieser Stoffe in einem nachfolgenden Reinigungsschritt nicht erforderlich.

Da in wäßrigem Dispersionsmedium gearbeitet wird, gestaltet sich der Herstellungsprozeß des polymeren Grundkörpers als besonders kostengünstig. Vorzugsweise wird der Polymerisationsschritt in Form eines Batch-Prozesses durchgeführt, dessen Up-Scaling keine Schwierigkeiten bereitet, da Schwankungen in der Temperaturführung sowie die gewählte Rührtechnik nur einen geringen Einfluß auf die Eigenschaften des Endproduktes haben. Je nach gewählter Monomer- und Initiatorkonzentration des Reaktionsansatzes weisen die Polymerteilchen nach der Emulsionspolymerisation eine Teilchengröße von vorzugsweise 10 bis 1000 nm und eine Oberflächenladung von 0,1 bis 10 C/g Polymer auf.

Die erhaltenen Polymersuspensionen sind auch bei Raumtemperatur über mehrere Monate lagerfähig, so daß irgendwelche Agglomerationen nicht zu beobachten sind. Die weiteren Vorteile dieser polymeren Trägermaterialien sind ihre hohe Stabilität in biologischen Medien und geringe Toxizität im gewünschten Anwendungsbereich.

Vor der Umsetzung der Polymersuspensionen mit den Nucleinsäuren werden gemäß einer bevorzugten Ausführungsform sowohl die Polymersuspensionen als auch die Nucleinsäuren bspw. durch Zentrifugation oder Diafiltration aufgereinigt. Bei der bevorzugten Diafiltration kann auf die üblichen Dialyseverfahren und Membranen zurückgegriffen werden.

Darüber hinaus ist es im Rahmen der vorliegenden Erfindung noch möglich, daß man den Polymersuspensionen nach der Polymerisation Stabilisatoren in einer Menge von vorzugsweise 0,01 bis 5 Gew.-% bezogen auf das Gewicht der Suspension zusetzt, um auf diese Weise eine zusätzliche sterische Stabilisierung der Polymersuspension zu erreichen. Als Stabilisatoren werden hierzu vorzugsweise biologisch inerte, nicht-ionische Blockcopolymere mit hydrophoben und hydrophilen Anteilen eingesetzt. Beispiele für solche Stabilisatoren sind Poloxamere oder Poloxamine.

Die eigentliche Umsetzung der Polymersuspensionen mit den Nucleinsäuren entsprechend der zweiten Stufe des erfindungsgemäßen Verfahrens erfolgt vorzugsweise bei Temperaturen von 10 bis 30 °C und einem pH-Wert < 11, wobei allerdings die zur Anbindung verwendeten Nucleinsäuren in deprotonierter Form vorliegen sollten. Als Nucleinsäuren kommen hierbei bspw. Desoxyribonucleotide, Ribonucleotide oder chemisch modifizierte Desoxyribonucleotide und Ribonucleotide mit 7 bis 40 Nucleotideinheiten in Frage. Darüber hinaus können jedoch auch ohne weiteres Plasmide als Nucleinsäuren verwendet werden.

Die bei der Umsetzung entstehenden erfindungsgemäßen Polymernanopartikel-Nucleinsäure-Konjugate, welche durch den Nucleinsäureanteil eine gewisse negative Überschußladung aufweisen, können gemäß einer bevorzugten Ausführungsform in einer Art Sandwich-Komplex zusätzlich mit Peptiden, Proteinen mit einem isoelektrischen Punkt > 7 oder Polyethylenimin modifiziert werden. Die Beladungskapazität des Trägermaterials mit Nucleinsäure bzw. Peptiden oder Proteinen wird durch den Zusatz von sterischen Stabilisatoren nicht beeinflußt. Die effektiv gebundene Nucleinsäure- bzw. Peptid- oder Proteinmenge kann durch Abzentrifugation der Polymernanopartikel-Nucleinsäure-Konjugate und Messung der im Überstand vorhandenen überschüssigen ungebundenen Substratmenge leicht bestimmt werden.

Die erfindungsgemäßen Polymernanopartikel-Nucleinsäure-Konjugate eignen sich aufgrund ihrer biologischen Aktivität hervorragend zum Gentransfer sowie zur Kontrolle der Genexpression.

Als Testsystem zur Untersuchung der Effizienz der erfindungsgemäßen Polymernanopartikel-Nucleinsäure-Konjugate dienten Rattenhepatozyten, deren Multiple Drug Resistance-(mdr-) Gen durch den Einsatz von Antisense Oligonucleotiden inhibiert werden sollte. Überraschenderweise kann die Konzentration an anti-mdr Phosphorthioat-Oligonucleotiden bei den Konjugaten im Vergleich zu den freien Oligonucleotiden um den Faktor 10 herabgesetzt werden, um die gleiche Wirkung zu erzielen. Noch deutlicher wird der Unterschied bei Verwendung von nucleaselabilen Phosphordiester-Oligonucleotiden, die nur im Falle der erfindungsgemäßen Konjugate überhaupt eine Wirkung zeigen, da sie in der Konjugatform vor enzymatischem Abbau geschützt sind. Aufgrund ihrer höheren Spezifität und Biokompatibilität stellen jedoch gerade die unmodifizierten, natürlichen DNA-Fragmente eine interessante Variante in der Antisense-Strategie dar.

Auch die erfindungsgemäßen Polymernanopartikel-Nucleinsäure-Konjugate in Form von Plasmiden eignen sich hervorragend zum Gentransfer. Zur Untersuchung der Beladungskapazität und Wirksamkeit zum Gentransfer wurden Plasmide, die β-Galactosidase-Gene als leicht nachweisbare Expressionskontrollen enthalten, eingesetzt. Die Beladungskapazität (in µg DNA/mg Polymer) lag etwa um den Faktor 4 höher als bei kurzen Oligonucleotid-Fragmenten.

Die besonderen Vorteile der erfindungsgemäßen Polymernanopartikel- Nucleinsäure-Konjugate bestehen im wesentlichen darin, daß bei der Herstellung weder die Verwendung von ionischen Comonomeren noch der Einsatz von hydrophoben Kationen als Ladungsträger erforderlich ist, da die negativ geladenen Nucleinsäuren direkt von den geladenen Endgruppen auf der Partikeloberfläche elektrostatisch gebunden werden. Nebenwirkungen in biologischen Systemen, insbesondere bei Zellkulturexperimenten, die häufig bei Verwendung von biologisch abbaubaren Trägermaterialien oder adsorptiv gebundenen Hilfsstoffen auftreten, sind im vorliegenden Fall minimal, da außer den gebundenen Nucleinsäuren bzw. Peptiden keine biologisch wirksamen Substanzen freigesetzt werden. Wie Bioverträglichkeitstests an Rattenhepatozyten zeigen, liegt die LDH-Ausschüttung als Assay für die Zytotoxizität bspw. für Polystyrol bei Konzentrationen von 1 g Polymer/l Inkubationsmedium nach 20 Stunden nur um 5 % höher als bei der unbehandelten Kontrolle.

Die erfindungsgemäßen Polymernanopartikel-Nucleinsäure-Konjugate und deren Komplexe mit basischen Peptiden oder Proteinen stellen somit ein neues und effizientes System zur *in vitro*-Kontrolle der Genexpression (Antisense-Strategie) dar. Ferner können sie in vielfältiger Weise als Vektoren zur Gentransfektion eingesetzt werden. So lassen sich durch Variation der Größe, Art und Oberflächenladung des polymeren Nanopartikel-Grundkörpers durch Wahl der wirksamen Nucleinsäure-Komponente und deren Derivatisierung sowie durch die Modifikation der Oberfläche mittels basischer Peptide oder Polyethylenimin die Polymernanopartikel-Nucleinsäure-Systeme auf die jeweiligen Anforderungen der biologischen Testsysteme anpassen.

Die nachfolgenden Beispiele sollen die Erfindung näher veranschaulichen.

### Beispiele

### Beispiel 1

In einem 100 ml Rundkolben, versehen mit KPG-Rührer, Rückflußkühler, Argoneinlaß- und -auslaßvorrichtungen werden 40 ml Reinstwasser und 2,5 ml zuvor unter Schutzgas (Argon) frisch destilliertes Styrol gegeben und unter Durchleiten eines Argonstroms intensiv durchmischt. Dem Ansatz werden 50 mg 2,2'-Azobis(2-amidinopropan)dihydrochlorid (AIBA), gelöst in 5 ml H₂O, zugefügt und das Reaktionsgefäß in ein auf 80 °C temperiertes Ölbad getaucht und bei einer Rührerdrehzahl von 360 U/Min. gerührt. Nach ca. 10 Min. ist eine milchig weiße Trübung des Reaktionsansatzes zu erkennen. Nach 24 Stunden wird auf Raumtemperatur abgekühlt. Man erhält eine Polymersuspension mit einem Feststoffgehalt von 42 g/l und einem Teilchendurchmesser von 400 bis 500 nm, die eine weitgehend monodisperse Größenverteilung der Partikel aufweist. Das Material wird über einen Zeitraum von 7 Tagen mittels einer Dialysemembran (SpectraPor; MWCO: 10000; Fa. Roth, Karlsruhe) gegen Reinstwasser dialysiert, um im Dispersionsmedium gelöstes Polymer und Restmonomerspuren zu entfernen. Das aufgereinigte Produkt weist einen Feststoffgehalt von 40 g/l und eine konduktometrisch bestimmte Partikelladung von 750 mC/g Polymer auf.

Nach der Phosphoramidit-Methode synthetisch hergestelltes Phosphorothioat-Oligodesoxynucleotid 20mer der Sequenz 5' CCC TGC TCC CCC CTG GCT CC 3' (ODN-1; M_{W}: 6207 g/mol) wird mittels präparativer HPLC aufgereinigt und nach Abspaltung der Dimethoxytritylschutzgruppe mittels einer sterilen Dialysemembran (SpectraPor CE; MWCO: 500; Fa. Roth, Karlsruhe) gegen Reinstwasser über einen Zeitraum von 7 Tagen dialysiert.

200 µl der Polymersuspension (8 mg Polymer) werden mit 100 ng (16 nmol) Phosphorothioat-Oligonucleotid (ODN-1), gelöst in Reinstwasser, über einen Zeitraum von 12 bis 24 Stunden inkubiert. Man erhält ein Polymernanopartikel-Oligonucleotid-Konjugat mit einem Nucleinsäureanteil von 1,7 µmol Oligonucleotid/g Polymer (10,6 mg Nucleinsäure/g Polymer).

Die Bestimmung der Beladungskapazität erfolgt nach dem nachfolgend beschriebenen Verfahren:

Nach der Inkubation der Nanopartikel mit der Nucleinsäure wird das Konjugat abzentrifugiert (14 000 g, 2 x 45 Min.) und die Menge an ungebundener Substanz im Überstand mittels UV-Messung bestimmt. Die adsorptiv gebundene Substanzmenge wird durch die Differenz zur Ausgangsmenge an Nucleinsäure bzw. Peptid berechnet.

### Beispiel 2

Der Polymerisationsansatz und die Aufreinigung des Produktes erfolgt analog zu Beispiel 1. Dabei werden jedoch 1,5 ml Styrol eingesetzt. Man erhält eine Polymersuspension mit einem Feststoffgehalt von 23 g/l. Die Partikel weisen Teilchendurchmesser von 150 bis 400 nm auf. Nach der Dialyse liegt der Feststoffgehalt bei 22 g/l und die Partikelladung bei 1080 mC/g.

Nach Inkubation von 100 µl der Polymersuspension (2,2 mg Polymer) mit 23,8 ng (5,3 nmol) Phosphordiester-Oligonucleotid 15mer der Sequenz 5' TTC TTG TCT GCT CTT 3' (ODN-2; M_{W}: 4491 g/mol) analog zu Beispiel 1 erhält man ein Polymernanopartikel-Oligonucleotid-Konjugat mit einem Nucleinsäureanteil von 2,0 µmol Oligonucleotid/g Polymer (8,9 mg Nucleinsäure/g Polymer).

### Beispiel 3

Analog zu Beispiel 1 wird zu 80 ml H₂O und 3 ml Styrol eine Lösung von 118 mg 2,2'-Azobis(2-(2-imidazolin-2-yl)propan)dihydrochlorid (AIBI) in 10 ml Wasser gegeben und bei 80°C über einen Zeitraum von 24 Stunden durchpolymerisiert. Die erhaltene Polymersuspension hat einen Feststoffgehalt von 23,9 g/l und besteht aus Partikeln mit einem mittleren Teilchendurchmesser von 150 bis 200 nm, die eine weitgehend monodisperse Größenverteilung aufweist. Der Feststoffgehalt beträgt nach der Dialyse über einen Zeitraum von 21 Tagen 22,7 g/l und die Partikelladung 930 mC/g. Da die mit AIBI hergestellten Polymerpartikel eine höhere Oberflächenladung und eine höhere Beladungskapazität aufweisen, bilden solche Partikel die besten Konjugate mit Nucleinsäuren und stellen daher nach gegenwärtigem Kenntnisstand der Erfinder die beste Ausführungsform der Erfindung dar.

Die Toxizität der Polymersuspension (stabilisiert mit einer wäßrigen Lösung von 0,1 % (w/v) Poloxamer 338 (Fa. ICI Chemicals; Manchester, UK) ; Dichte nach der Stabilisierung: 9,56 g/l) bei Rattenhepatozyten, bestimmt über die LDH-Ausschüttung, beträgt nach 20 Stunden 81 Einheiten im Gegensatz zu 77 Einheiten der unbehandelten Kontrolle (1 g Polymer/l Inkubationsmedium).

Nach Inkubation von 200 µl der stabilisierten Polymersuspension (4,5 mg Polymer) mit 72,7 ng (16,2 nmol) ODN-2 analog dem Verfahren zu Beispiel 1 erhält man ein Polymernanopartikel-Oligonucleotid-Konjugat mit einem Nucleinsäureanteil von 3,0 µmol Oligonucleotid/g Polymer (13,5 mg Nucleinsäure/g Polymer).

### Beispiel 4

Die Inkubation von 200 µl der aufgereinigten und stabilisierten Polymersuspension aus Beispiel 3 mit 76,4 ng (16,2 nmol) des Phosphorothioat-Oligodesoxynucleotids ODN-3 (M_{W}: 4716 g/mol; Sequenz analog zu ODN-2 in Beispiel 2) ergibt ein Polymernanopartikel- Oligonucleotid-Konjugat mit einem Nucleinsäureanteil von 3,1 µmol Oligonucleotid/g Polymer (14,6 mg Nucleinsäure/g Polymer; Bestimmung analog zu Beispiel 1).

Die Inhibition der mdr-Genexpression der Polymernanopartikel-ODN-3-Konjugate wird mittels mRNA-Bestimmung (Northern Plot) und mdr-Protein-Bestimmung (Western Plot) an Rattenhepatozyten untersucht. Die Polymernanopartikel-Oligonucleotid-Konjugate werden in Konzentrationen von 0,032, 0,096, 0,32 und 0,96 g Polymer/l Inkubationsmedium (0,1, 0,3, 1 bzw. 3 µmol ODN-3/l Inkubationsmedium) eingesetzt. Bei einer Konzentration von 1 µmol ODN-3/l Inkubationsmedium kann der mRNA-Gehalt um 90 % und der Protein-Gehalt um 50 % verringert werden. Mit freiem Oligonucleotid wird der gleiche Inhibitionseffekt erst bei einer Konzentration von 10 µmol ODN-3/l Inkubationsmedium erzielt. Kontrolloligonucleotide (ODN-4; Sequenz: 5' CCT GTT GTT TTC TCT 3' bzw. ODN-5; Sequenz: 5' AAG AGC AGA CAA GAA 3') zeigen sowohl in freiem Zustand als auch in der Konjugatform mit Nanopartikeln bei den verwendeten Konzentrationen keinerlei Effekte.

### Beispiel 5

Zur Untersuchung der Ansatzgröße der Polymerisation auf die Eigenschaften des Endproduktes wurde der Reaktionsansatz von Beispiel 3 um das Fünffache erhöht. Die Charakterisierung des aufgereinigten Materials ergab in Bezug auf Teilchengröße, Größenverteilung, Oberflächenladung und Beladungskapazität an Nucleinsäure keine nennenswerten Unterschiede zu den Ergebnissen aus Beispiel 3 (Feststoffgehalt nach der Dialyse: 23,8 mg; Partikelladung: 1 020 mC/g Polymer; Nucleinsäureanteil: 3,4 µmol ODN-3/g Polymer bzw. 16,0 mg ODN-3/g Polymer; Bestimmung analog zu Beispiel 1).

### Beispiel 6

Bei Inkubation von 50 µl der stabilisierten Polymersuspension aus Beispiel 3 mit 22 µg pcMVβ-Plasmid gelöst in 100 µl Reinstwasser erhält man ein Polymernanopartikel-Plasmid-Konjugat mit einem Nucleinsäureanteil von 42 mg Nucleinsäure/g Polymer. Das Konjugat wird 12 bis 24 Stunden mit 2,6 µg (0,95 nmol) des Peptids Penetratin (Homeodomain-Sequenz; M_{W}: 2720,2 g/mol; Derossi, D., Joliot, A.H., Chassaing, G., Prochiantz, A., *J.Biol.Chem*. 1994, 269 (14), 10444-10450) inkubiert. Die Bestimmung der Beladungskapazität von Plasmid bzw. Peptid erfolgt analog zu Beispiel 1.

### Beispiel 7

Der Polymerisationsansatz entspricht dem in Beispiel 3, jedoch werden 2 ml Styrol eingesetzt. Das Rohprodukt besitzt einen Feststoffgehalt von 10,4 g/l und weist eine polydisperse Verteilung der Teilchendurchmesser von 50 bis 200 nm auf. Nach der Aufreinigung mittels Dialyse über einen Zeitraum von 10 Tagen beträgt der Feststoffgehalt 8,2 g/l und die Partikelladung 2190 mC/g.

## Patentansprüche

1. Verfahren zur Herstellung von biologisch aktiven Polymernanopartikel- Nucleinsäure-Konjugaten durch Polymerisation von vinylischen Monomeren mit einer geringen Wasserlöslichkeit in wäßriger Lösung und anschließender Umsetzung der entstehenden Polymersuspensionen mit den Nucleinsäuren, **dadurch gekennzeichnet, daß** man die Polymerisation der vinylischen Monomeren in Gegenwart von kationischen Radikalstartern und in Form einer emulgatorfreien Emulsionspolymerisation durchführt.

2. Verfahren nach Anspruch 1, **dadurch gekennzeichnet, daß** die vinylischen Monomere eine Wasserlöslichkeit von < 20 g/l aufweisen.

3. Verfahren nach den Ansprüchen 1 und 2, **dadurch gekennzeichnet, daß** als vinylische Monomere Styrol, Acrylsäure-Derivate, Methacrylsäure- Derivate oder Mischungen davon eingesetzt werden.

4. Verfahren nach den Ansprüchen 1 bis 3, **dadurch gekennzeichnet, daß** man kationische Radikalstarter mit basischen Endgruppen, wie z. B. 2,2'-Azobis(2-amidinopropan)di-hydrochlorid (AIBA) oder 2,2'-Azobis(2-(2-imidazolin-2-yl)propan)di-hydrochlorid (AIBI), verwendet.

5. Verfahren nach den Ansprüchen 1 bis 4, **dadurch gekennzeichnet, daß** man die Emulsionspolymerisation bei Temperaturen von 20 bis 100 °C durchführt.

6. Verfahren nach den Ansprüchen 1 bis 5, **dadurch gekennzeichnet, daß** die Polymerteilchen nach der Emulsionspolymerisation eine Teilchengröße von 10 bis 1000 nm aufweisen.

7. Verfahren nach den Ansprüchen 1 bis 6, **dadurch gekennzeichnet, daß** die Polymersuspensionen vor der Umsetzung mit Nucleinsäuren bspw. durch Zentrifugation oder Diafiltration aufgereinigt werden.

8. Verfahren nach den Ansprüchen 1 bis 7, dadurch gekennzeichnt, daß die Umsetzung der Polymersuspensionen mit den Nucleinsäuren bei Temperaturen von 10 bis 30 °C und einem pH-Wert < 11 erfolgt.

9. Verfahren nach den Ansprüchen 1 bis 8, **dadurch gekennzeichnet, daß** man als Nucleinsäuren Desoxyribonucleotide, Ribonucleotide oder chemisch modifizierte Desoxyribonucleotide und Ribonucleotide mit 7 bis 40 Nucleotid-Einheiten einsetzt.

10. Verfahren nach den Ansprüchen 1 bis 8, **dadurch gekennzeichnet, daß** man als Nucleinsäuren Plasmide verwendet.

11. Verfahren nach den Ansprüchen 1 bis 10, **dadurch gekennzeichnet, daß** den Polymersuspensionen nach der Polymerisation Stabilisatoren in einer Menge von 0,01 bis 5 Gew.-% bezogen auf das Gewicht der Suspension zugesetzt werden.

12. Verfahren nach Anspruch 11, **dadurch gekennzeichnet, daß** man als Stabilisatoren nichtionische Block-Copolymere mit hydrophoben und hydrophilen Anteilen einsetzt.

13. Verfahren nach Anspruch 12, **dadurch gekennzeichnet, daß** man als nichtionische Block-Copolymere Poloxamere oder Poloxamine verwendet.

14. Verfahren nach den Ansprüchen 1 bis 13, **dadurch gekennzeichnet, daß** die Polymernanopartikel-Nucleinsäure-Konjugate mit Peptiden, Proteinen mit einem isoelektrischen Punkt > 7 oder Polyethylenimin modifiziert werden.

15. Polymernanopartikel-Nucleinsäure-Konjugate, erhältlich nach einem Verfahren gemäß einem der vorhergehenden Ansprüche.

16. Verwendung der Polymernanopartikel-Nucleinsäure-Konjugate gemäß Anspruch 15 zum in vitro Gentransfer.

17. Verwendung der Polymernanopartikel-Nucleinsäure-Konjugate gemäß Anspruch 15 zur Kontrolle der in vitro Genexpression.

## Claims

1. Process for the production of biologically active polymer nanoparticle-nucleic acid conjugates by polymerisation of vinyl monomers having a low water solubility in aqueous solution and then reaction of the resulting polymer suspensions with the nucleic acids, **characterised in that** polymerisation of the vinyl monomers is carried out in the presence of cationic free-radial initiators and in the form of emulsifier-free emulsion polymerisation.

2. Process according to claim 1, **characterised in that** the vinyl monomers have a water solubility of< 20 g/litre.

3. Process according to claims 1 and 2, **characterised in that** styrene, acrylic acid derivatives, methacrylic acid derivatives or mixtures thereof are used as vinyl monomers.

4. Process according to claims 1 to 3, **characterised in that** cationic free-radical initiators having basic end groups, such as for example 2,2'-azobis(2-amidinopropane)di-hydrochloride (AIBA) or 2,2'-azobis(2-(2-imidazoline-2-yl)propane)di-hydrochloride (AIBI), are used.

5. Process according to claims 1 to 4, **characterised in that** emulsion polymerisation is carried out at temperatures from 20 to 100°C.

6. Process according to claims 1 to 5, **characterised in that** the polymer particles after emulsion polymerisation have a particle size of 10 to 1,000 nm.

7. Process according to claims 1 to 6, **characterised in that** the polymer suspensions are purified before the reaction with nucleic acids, for example by centrifuging or diafiltration.

8. Process according to claims 1 to 7, **characterised in that** the reaction of the polymer suspensions with the nucleic acids takes place at temperatures from 10 to 30°C and a pH value < 11.

9. Process according to claims 1 to 8, **characterised in that** deoxyribonucleotides, ribonucleotides or chemically modified deoxyribonucleotides and ribonucleotides having 7 to 40 nucleotide units are used as nucleic acids.

10. Process according to claims 1 to 8, **characterised in that** plasmids are used as nucleic acids.

11. Process according to claims 1 to 10, **characterised in that** stabilisers in a quantity of 0.01 to 5 wt.%, based on the weight of the suspension, are added to the polymer suspensions after polymerisation.

12. Process according to claim 11, **characterised in that** non-ionic block copolymers having hydrophobic and hydrophilic portions are used as stabilisers.

13. Process according to claim 12, **characterised in that** poloxamers or poloxamines are used as non-ionic block copolymers.

14. Process according to claims 1 to 13, **characterised in that** the polymer nanoparticle-nucleic acid conjugates are modified with peptides, proteins having an isoelectric point > 7 or polyethyleneimine.

15. Polymer nanoparticle-nucleic acid conjugates which can be obtained by a process according to one of the preceding claims.

16. Use of the polymer nanoparticle-nucleic acid conjugates according to claim 15 for in vitro gene transfer.

17. Use of the polymer nanoparticle-nucleic acid conjugates according to claim 15 for the control of in vitro gene expression.

## Revendications

1. Procédé de production de conjugués biologiquement actifs d'acides nucléiques et de nanoparticules polymères par polymérisation en solution aqueuse de monomères vinyliques de faible solubilité dans l'eau et transformation ultérieure des suspensions de polymère formées avec les acides nucléiques, **caractérisé en ce que** l'on effectue la polymérisation des monomères vinyliques en présence d'initiateurs de radicaux cationiques et sous la forme d'une polymérisation en émulsion exempte d'émulsifiant.

2. Procédé selon la revendication 1, **caractérisé en ce que** les monomères vinyliques ont une solubilité dans l'eau de < 20 g/l.

3. Procédé selon les revendications 1 et 2, **caractérisé en ce que** le styrène, des dérivés d'acide acrylique, des dérivés d'acide méthacrylique ou leurs mélanges sont utilisés comme monomère vinylique.

4. Procédé selon les revendications 1 à 3, **caractérisé en ce que** l'on utilise des initiateurs de radicaux cationiques avec des groupes terminaux basiques, comme par exemple le 2,2'-azobis (2-amidinopropane) di-hydrochlorure (AIBA) ou le 2,2'-azobis (2-(2-imidazolin-2-yl) propane) di-hydrochlorure (AIBI).

5. Procédé selon les revendications 1 à 4, **caractérisé en ce que** l'on effectue la polymérisation en émulsion à des températures de 20 à 100°C.

6. Procédé selon les revendications 1 à 5, **caractérisé en ce que** les particules de polymère après la polymérisation en émulsion présentent une taille de particules de 10 à 1000 nm.

7. Procédé selon les revendications 1 à 6, **caractérisé en ce que** les suspensions de polymère sont purifiées par exemple par centrifugation ou diafiltration avant la transformation avec des acides nucléiques.

8. Procédé selon les revendications 1 à 7, **caractérisé en ce que** la transformation des suspensions de polymère avec les acides nucléiques a lieu à des températures de 10 à 30°C et à une valeur de pH < 11.

9. Procédé selon les revendications 1 à 8, **caractérisé en ce que** l'on utilise comme acides nucléiques des désoxyribonucléotides, des ribonucléotides ou des désoxyribonucléotides et ribonucléotides chimiquement modifiés avec de 7 à 40 unités nucléotides.

10. Procédé selon les revendications 1 à 8, **caractérisé en ce que** l'on utilise des plasmides comme acides nucléiques.

11. Procédé selon les revendications 1 à 10, **caractérisé en ce que** l'on ajoute aux suspensions de polymère, après la polymérisation, des stabilisateurs en une quantité de 0,01 à 5 % en poids par rapport au poids de la suspension.

12. Procédé selon la revendication 11, **caractérisé en ce que** l'on utilise comme stabilisateurs des copolymères à blocs non ioniques avec des parties hydrophobes et hydrophiles.

13. Procédé selon la revendication 12, **caractérisé en ce que** l'on utilise des poloxamères ou des poloxamines comme copolymères à blocs non ioniques.

14. Procédé selon les revendications 1 à 13, **caractérisé en ce que** les conjugués nanoparticules polymères - acides nucléiques sont modifiés avec des peptides, des protéines ayant un point isoélectrique > 7 ou des polyéthylèneimines.

15. Conjugués nanoparticules polymères - acides nucléiques capables d'être obtenus par un procédé selon l'une des revendications précédentes.

16. Utilisation des conjugués nanoparticules polymères - acides nucléiques selon la revendication 15 pour le transfert de gènes in vitro.

17. Utilisation des conjugués nanoparticules polymères - acides nucléiques selon la revendication 15 pour le contrôle de l'expression de gènes in vitro.
